# EUROPEAN PATENT APPLICATION

(11) **EP 4 809 930 A1**
(43) Date of publication of application: **23.09.2026**
(21) Application number: 25164191.6
(22) Date of filing: 17.03.2025
(51) Int. Cl.: A61B 6/50, A61B 6/00, G06T 5/73, G06T 7/12

(54) **GENERATION OF ENHANCED CHEST X-RAY IMAGES**

(71) Applicant: Koninklijke Philips N.V., 5656 AE Eindhoven (NL)
(72) Inventor: PAUL, Soubhik, Eindhoven (NL)
(74) Representative: Philips Intellectual Property & Standards

(57) **Abstract**

Disclosed herein is a method of X-ray imaging. The method comprises receiving (200) a chest X-ray image (124, 124', 124"). The method further comprises registering (202) a pulmonary blood density mask (122) to the chest X-ray image. The method further comprises generating (204) an enhanced chest X-ray image (132, 132') by compensating for a spatially dependent blood density (602, 604, 700, 702, 704) in the chest X-ray image using the registered pulmonary blood density mask (128). The method comprises further providing (206) the enhanced chest X-ray image.

## Description

### FIELD OF THE INVENTION

The invention relates to X-ray imaging, in particular to X-ray imaging of the thoracic region.

### BACKGROUND OF THE INENTION

X-ray imaging of the chest is a diagnostic technique that relies on the differential absorption of X-rays by various tissues to produce a two-dimensional projection of a subject's internal anatomy. Hard tissue such as bones absorb more X-rays and appear prominently, while soft tissues, including the lungs, allow more X-rays to pass through, resulting in lower contrast. The visualization of lung tissue is particularly challenging due to its low radiodensity, which makes distinguishing pathological changes from normal anatomical variations or the identification of various types of lesions difficult.

United States patent application publication US2016210739A1 relates to lung measurement. In order to provide enhanced information about a patient that facilitates further assessment steps, 2D X-ray image data of a patient's chest is provided, and the image data is segmented to identify lung structures to provide segmented image data separated from un-segmented areas. Further, spatial lung volume information is extracted from the image data using the segmented image data derived from the image data. Still further, lungs symmetry information is determined using the extracted spatial lung volume information. Finally, the lungs symmetry information is provided to a user. A 2D X-ray image data of a patient's chest is provided and a lungs mask image is formed after the step of segmenting the input image data. Then, the lungs mask image is used to define areas, within which a predetermined adaptation is applied to the original 2D X-ray image data producing a thorax mask image. Next, left and right images are provided showing the left and the right spatial lungs volume information of the regions defined originally by the lungs mask image. Finally, based on the spatial lungs volume information, lungs symmetry information or total lung volumes may be calculated and provided.

### SUMMARY OF THE INVENTION

The invention provides for a method of X-ray imaging, a computer program product, and a medical system in the independent claims. Embodiments are given in the dependent claims.

In one aspect, a method of X-ray imaging is disclosed. The method comprises receiving a chest X-ray image. The method further comprises registering a pulmonary blood density mask to the chest X-ray image. The method further comprises generating an enhanced chest X-ray image by compensating for a spatially dependent blood density in the chest X-ray image using the registered pulmonary blood density mask. The method further comprises providing the enhanced chest X-ray image.

In another aspect, a computer program product is disclosed. The computer program product comprises a computer-readable storage medium having machine-executable instructions embodied therewith. The machine-executable instructions are configured to implement an example of the method.

In another aspect, a medical system is disclosed. The medical system comprises a memory storing machine-executable instructions and a pulmonary blood density mask. The medical system further comprises a computational system. Execution of the machine-executable instructions causes the computational system to receive a chest X-ray image. Execution of the machine-executable instructions further causes the computational system to register a pulmonary blood density mask to the chest X-ray image. Execution of the machine-executable instructions further causes the computational system to generate an enhanced chest X-ray image by compensating for a spatially dependent blood density in the chest X-ray image using the registered pulmonary blood density mask. Execution of the machine-executable instructions further causes the computational system to provide the enhanced chest X-ray image.

### BRIEF DESCRIPTION OF THE DRAWINGS

In the following preferred embodiments of the invention will be described, by way of example only, and with reference to the drawings in which:
Fig. 1 illustrates an example of a medical system.
Fig. 2 shows a flow chart which illustrates a method of using the medical system of Fig. 1.
Fig. 3 illustrates a further example of a medical system.
Fig. 4 shows a flow chart which illustrates a method of using the medical system of Fig. 1.
Fig. 5 illustrates an example of a cloud computing based medical system.
Fig. 6 illustrates the change in blood density in the lungs due to pulmonary blood circulation.
Fig. 7 illustrates the change in blood density due to gravitational effects.
Fig. 8 illustrates the boundary of the pulmonary blood density map.
Fig. 9 illustrates the filling of the pulmonary blood density map with parabolas.
Fig. 10 shows a plot of equation 2
Fig. 11 shows a greyscale rendering of the pulmonary blood density map.
Fig. 12 illustrates the generation of an enhanced chest X-ray image.
Fig. 13 shows a chest X-ray image that shows ground glass opacity lesions.
Fig. 14 shows an example of a chest X-ray image that shows a pneumothorax.
Fig. 15 shows a plot of equation 3.
Fig. 16 illustrates pulmonary cavity edge enhancement in a chest X-ray image.
Fig. 17 shows an edge detection algorithm applied to the edge enhanced image of Fig. 16.
Fig. 18 shows the result of connected component analysis applied to the edge detection image of Fig. 17.
Fig. 19 illustrates the identification of connected components in the chest X-ray using the connected component analysis of Fig. 18
Fig. 20 shows two pulmonary cavity candidates identified from the connected components of Fig. 19
Fig. 21 illustrates the use of a frequency domain sinogram to detect circular objects in a chest X-ray.
Fig. 22 illustrates the use of a frequency domain sinogram to detect circular non-objects in a chest X-ray.
Fig. 23 shows several chest X-ray images with pulmonary cavity candidates identified.

### DETAILED DESCRIPTION OF EMBODIMENTS

Like numbered elements in these figures are either equivalent elements or perform the same function. Elements which have been discussed previously will not necessarily be discussed in later figures if the function is equivalent.

In one example, a method of X-ray imaging is disclosed. The method of X-ray imaging comprises receiving a chest X-ray image. The chest X-ray image may for example be acquired by X-raying the chest region of a subject. The method of X-ray imaging further comprises registering a pulmonary blood density mask to the chest X-ray image. The pulmonary blood density mask is used to model the blood density in the chest or lung region. The pulmonary blood density mask may depend upon whether the subject is sitting up or laying down in different examples. When the subject is sitting up then there may be a gravitational effect which adjusts the blood density as a function of height. The pulmonary blood density may also be dependent upon the distribution of arteries and veins within the lungs. The pulmonary blood density mask may for example be modeled or it may use predetermined values.

The method further comprises generating an enhanced chest X-ray image by compensating for a spatially dependent blood density in the chest X-ray image using the registered pulmonary blood density mask. The spatially dependent blood density may be the blood density projected in the direction of travel of the X-rays when the chest X-ray image was acquired. An increase in the blood density may result in an increase in the attenuation of X-rays when the chest X-ray image was acquired. By compensating for the blood density, other details in the chest X-ray may become more pronounced or visible. The method further comprises providing the enhanced chest X-ray image.

The enhanced chest X-ray image may have a variety of benefits. The removal of the effect of the pulmonary blood density may be useful in providing images which may be analyzed using algorithms or neural networks more effectively. For example, the greater detail in the chest X-ray images may enable better automatic segmentation or analyses of the chest X-ray images. Additionally, the quality of the chest X-ray image may improve as more anatomical structures are visible in general.

In another example, generating the enhanced chest X-ray image by compensating for the spatially dependent blood density in the chest X-ray image using the registered pulmonary blood density mask comprises performing pixel-wise multiplication between the pulmonary blood density mask and the chest X-ray image. For example, the blood density mask may be fit to a portion of the chest X-ray image and then the effect on individual pixels of the chest X-ray image can be accounted for. There may be a one-to-one overlap between the pixels of the chest X-ray image or the pulmonary blood density mask, in which case the pixels can simply be multiplied by each other. In other cases, the pulmonary blood density mask may be fit or adjusted in size to fit the chest X-ray image during registration. The pixel-wise multiplication could for example be performed by calculating the average value of the pulmonary blood density mask that overlaps individual pixels. As with an increase in the pulmonary blood density mask, the effect of multiplying may have the effect of making the pixel brighter. This event counteracts for the attenuation of the X-rays by the blood in the lungs. This may provide an effective means of compensating for the absorption of X-rays when imaging the lungs.

In another example, the compensation for the spatially dependent blood density in the chest X-ray image further comprises rescaling the enhanced chest X-ray image to an original bit range of the chest X-ray image. For example, simply going and multiplying the blood density mask times the chest X-ray image may result in an image which exceeds the bit range or is too bright. Essentially the entire image can be rescaled. For example, the maximum value after the pixel-wise multiplication may be chosen as the highest value for the original bit range of the chest X-ray image. The other values may for example be scaled linearly accordingly. This may have the effect of producing an enhanced chest X-ray image that is more compatible with the original chest X-ray image.

In another example, the pulmonary blood density mask is descriptive of a pulmonary blood density due to pulmonary blood circulation. As was mentioned before, the concentration of veins and arteries affect the blood density. Various models or mathematical models may be used to model the blood density according to the density of the arteries and veins. This can be done using a numerical approximation, for example using a model, or in other cases, an anatomical model which calculates values according to the actual densities of arteries and veins may be used. Doing this may for example result in a pulmonary blood density mask which is more accurate and results in production of a better enhanced chest X-ray image.

In another example, the pulmonary blood density mask is compensated for gravitational forces on the pulmonary blood circulation. If the subject is laying down then the gravitational forces will not have an effect on the pulmonary blood density. However, if the X-ray is taken when the subject is in a standing or sitting position, this produces a higher blood density in the lower range of the lungs than at the top of the lungs.

In another example, the pulmonary blood density mask comprises an array of predetermined values. For example, the pulmonary blood density map may be an array which has values which represent the spatially dependent blood density. This may be calculated using empirical measurements, using anatomical atlases or other models.

In another example, the pulmonary blood density map has a boundary defined by a closed polynomial. This may be beneficial because it may provide a means of analytically determining the pulmonary blood density map.

In another example, the pulmonary blood density map has weighting values defined by a mapping fit to parabolas passing through a central point of the pulmonary blood density map. The use of parabolas provide for an effective means of modeling the arteries and veins in the lungs.

In another example, the mapping is further adjusted using a gravitational factor. This may be a constant gravitational factor or the mapping may be divided into a number of discreet regions. This may have the example of making the pulmonary blood density map more accurate when the subject is in a sitting or standing position.

In another example, the method further comprises identifying lung cavities in the enhanced chest X-ray using a pattern recognition algorithm. The chest cavities may be indicative of tuberculosis which is a life threatening lung disease. The automatic recognition of the lung cavities may for example provide for better identification of this potentially life threatening condition.

In another example, the pattern recognition algorithm is configured to generate an edge detection image by applying an edge detection algorithm to the enhanced X-ray image. The algorithm is further configured to detect enclosed regions in the edge detection image. For example, after the edge detection has been applied, it can then be detected if these are enclosed regions or are open. The algorithm further comprises identifying connected components using the enclosed regions. In this, various enclosed regions may be detected if they are connected and form a single structure. The edge detection algorithm is further configured to identify pulmonary cavity candidates by applying a pulmonary cavity detection algorithm to the connected components. After a connected component has been identified the pulmonary cavity detection algorithm then determines if it is a pulmonary cavity candidate or not. Typically this will be performed by identifying if the pulmonary cavity candidate is circular or more or less circular. The method then configures providing the pulmonary cavity candidates. This may have the advantage of more accurately identifying pulmonary cavity candidates automatically.

In another example, the pulmonary cavity detection algorithm is a trained pattern recognition neural network. For example, a classification neural network or a convolutional neural network which is configured to put a bounding box on an image may be used to check individual enclosed regions to see if they are circular or not. A U-net could also be trained to segment images to identify circular lesions. For example, a standard classifier algorithm may be trained using label data and then trained using a deep learning process.

In another example, the pulmonary cavity detection algorithm is a tomographic sinogram algorithm configured for identifying circular contours. This example may be beneficial because it provides a means of automatically identifying pulmonary cavity candidates without the need for training data.

The tomographic sinogram algorithm is configured to receive a connected component selected from the connected components. The tomographic sinogram algorithm is further configured to identify a center location of the connected component. For example, this may be a centroid of the connected component. The tomographic sinogram algorithm is further configured to calculate an angular sinogram of the connected component which determines the density of the connected component along a line through the center location as a function of the angular rotation of the line. Mathematically, this can be performed the same way in which a simulation of a CT image would be performed. For example, there is a line which is through the center location and then is rotated at various angles and the density within a particular distance of the line is used to calculate the overall density for this angular sinogram. The algorithm is further configured to transfer the angular sinogram to a frequency domain sinogram by spectrally transforming the angular sinogram. For example, a spectral transform such as a wavelet transform or a Fourier transform or other transform could be used.

The algorithm is further configured to calculate a standard deviation of the frequency domain sinogram. If the standard deviation is zero then the connected component would be circular. The standard deviation may then be used as a measure of how circular the particular connected component is. By setting a predetermined threshold the degree of how circular the connected component is can be assessed. The method therefore further comprises adding a connected component to the pulmonary cavity if the standard deviation is below the predetermined threshold. As was stated above, this provides an extremely accurate means of identifying lung cavities without the need for training data and also providing it in an analytic fashion which is able to be understood by a human.

In another example, the enhanced X-ray image is a pneumothorax detection image. This may for example be used to identify the separation of the lung from the lung's pleural cavity when a lung begins to collapse. This may be fairly difficult to identify in a normal X-ray image because of the poor contrast of spongy lung tissue. However, in the enhanced chest X-ray image the collapse of the lung is more visible.

In another example, the enhanced X-ray image is a ground glass opacity lesion detection image. A ground glass opacity lesion detection image is an image which is used to identify damage to the lungs due to COVID. The enhancement of the chest X-ray image according to examples described herein, it makes it easier to see the lesions caused by COVID.

In another example, the registering of the pulmonary blood density mask to the X-ray image comprises fitting the pulmonary blood density mask to anatomical landmarks in the chest X-ray image. There exist standard methods for identifying anatomical landmarks and these may for example be referenced to the pulmonary blood density mask. This may provide a very standardized means of fitting the pulmonary blood density mask as well as being able to do it without the need for training data.

In another example, the registering of the pulmonary blood density mask to the chest X-ray image comprises fitting the pulmonary blood density mask to a bounding box received in response to inputting this chest X-ray image into a trained neural network. For example, various enhanced X-ray images may be hand labeled as to the location of the lung. This may be used to train a neural network to put a bounding box on the enhanced X-ray images automatically and then the pulmonary blood density mask could just simple be fit to fit this bounding or rectangular box. A Region Proposal Neural Network (R-CNN), You Only Look Once (YOLO), or Single Shot MultiBox detector (SSD) neural network could, for example, be used to implement this.

In another example, the method further comprises controlling an X-ray system to acquire the test X-ray image. As was mentioned above, the subject may be in a prone position or may be sitting or standing. Depending upon the position of the subject, gravitational forces may or may not be taken into account in the pulmonary blood density mask. This method may provide for higher quality X-rays in which the details of the lung are better able to be observed.

In another aspect, a computer program product is disclosed. In this example, the computer program product comprises a computer-readable storage medium having machine-executable instructions stored on it. The machine-executable instructions are configured to implement a method as described above. The advantages of this have been previously discussed.

In another aspect, there is an example of a medical system. The medical system comprises a memory storing machine-executable instructions and a pulmonary blood density mask. The medical system further comprises a computational system. Execution of the machine-executable instructions causes the computational system to receive a chest X-ray image. Execution of the machine-executable instructions further causes the computational system to register a pulmonary blood density mask to the chest X-ray image. Execution of the machine-executable instructions further causes the computational system to generate an enhanced chest X-ray image by compensating for a spatially dependent blood density in the chest X-ray image using the registered pulmonary blood density mask. Execution of the machine-executable instructions further causes the computational system to provide the enhanced chest X-ray image. The advantages of this medical system have been previously disclosed in the context of the example and methods described above.

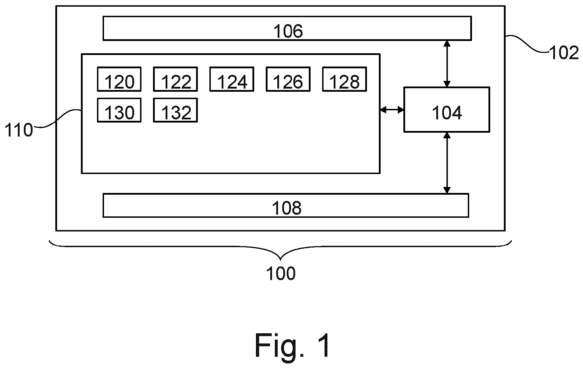

Fig. 1 illustrates an example of a medical system 100. This medical system 100 is shown as comprising a computer 102. The computer 102 may represent one or more computers or computing devices located at one or more locations. Multiple computers 102 could for example be connected by a network. The computer 102 is shown as comprising a computational system 104. The computational system 104 may comprise one or more computing cores or computational systems that are also located at one or more locations. The computational system 104 is connected to an optional hardware interface 106 and an optional user interface 108. The hardware interface 106 may for example be a network interface or other interface which allows the computational system 104 to communicate with other computing devices or control or communicate with other components of the medical system 100 if they are present. For example the hardware interface 106 could be used for controlling an X-ray system. The user interface 108 may provide a display for rendering X-ray images as well as providing a means for an operator to control the operation and function of the medical system 100.

The computational system 104 is further shown as being connected to a memory 110. The memory 110 is intended to represent various types of memory which may be accessible to the computational system 104. In some examples the memory 110 is a non-transitory storage medium. The memory 110 is shown as storing machine-executable instructions 120. The machine-executable instructions 120 enable the computational system 104 to perform such tasks as controlling other components such as an X-ray system as well as performing numerical and image processing tasks. The memory 110 is further shown as containing a pulmonary blood density map 122 which is used to provide a map which represents the blood density within the lung's pleural cavity or lung region of a subject. This is useful because the blood absorbs X-rays and will affect the contrast of an X-ray image. The memory 110 is further shown as containing a chest X-ray image 124 that has been acquired from a subject.

The memory 110 is further shown as containing an optional registration algorithm 126 that may be used to register the chest X-ray image 124 to the pulmonary blood density map 122. This could for example either use anatomical landmarks identified in a chest X-ray image 124 or the registration algorithm 126 could be a neural network that receives the chest X-ray image and outputs a bounding box, which the pulmonary blood density map 122 can be fit to. The memory 110 is shown as containing the registered pulmonary blood density map 128. The memory 110 is further shown as containing the optional product of the chest X-ray image and the registered pulmonary blood density map. Part of the registration process may also be determining the value of the pulmonary blood density map for the individual pixels of the chest X-ray image.

For example, when the pulmonary blood density map 128 is fit to the registration 128, the voxels may not line up precisely. In this case an average value for each voxel of the chest X-ray image may be calculated. In some cases, the product of the chest X-ray image and the registered pulmonary blood density map may be the enhanced chest X-ray image. However, it may be desirable to re-scale the image such that it has the original bit density. The memory 110 is further shown as containing the enhanced chest X-ray image 132. In this case, the enhanced chest X-ray image 132 has been a scaled version of the product image 130.

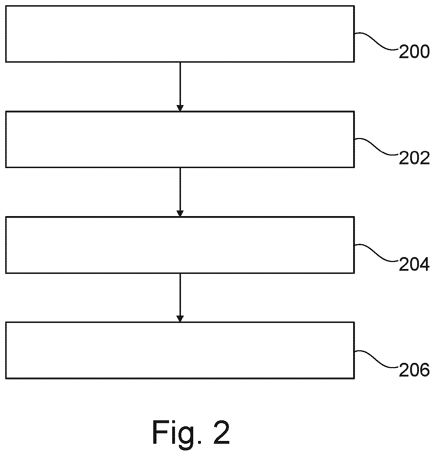

Fig. 2 shows a flowchart which illustrates a method of operating the medical system 100 of Fig. 1. In step 200 the chest X-ray image 124 is received. In step 202, the pulmonary blood density map 122 is registered to the chest X-ray image 124. This produces the registered pulmonary blood density map 128. In step 204, the enhanced chest X-ray image 132 is generated by compensating for a spatially dependent blood density in the chest X-ray image using the registered pulmonary blood density map 128. In step 206, the enhanced chest X-ray image 132 is provided. This may for example be performed by displaying it on the user interface 108 or it also may be provided by storing it in a database such as a DICOM database. The enhanced chest X-ray image 132 may also be provided by making it available or using it in further algorithms such as algorithms which are used to detect pneumothorax or a collapsed lung, a pulmonary cavity, or a ground glass opacity lesion.

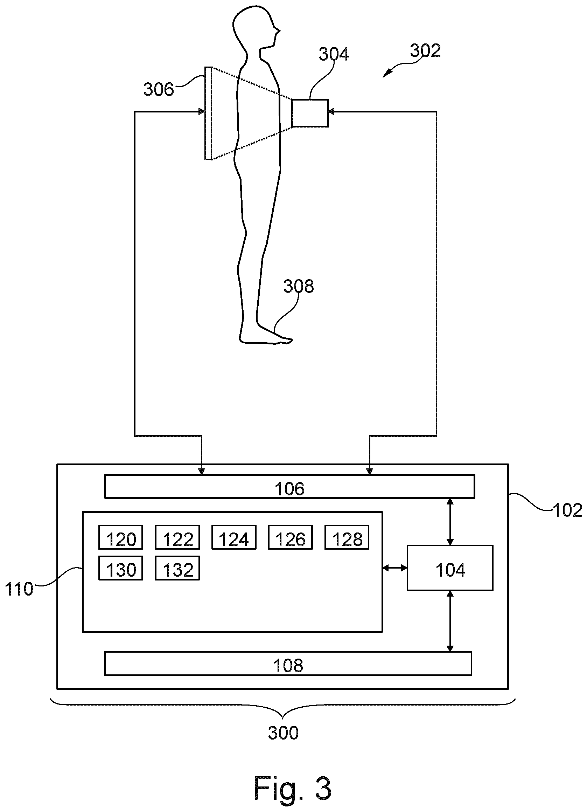

Fig. 3 shows a further example of a medical system 300. In this example the medical system 300 further comprises an X-ray system 302. The X-ray system 302 comprises an X-ray source 304 and an X-ray detector 306. A subject 308 is shown in a standing position and the X-ray system 302 is configured for X-raying a lung region of the subject 308. The hardware interface 106 is used to control the X-ray source 304 and read data from a spatially dependent X-ray detector 306.

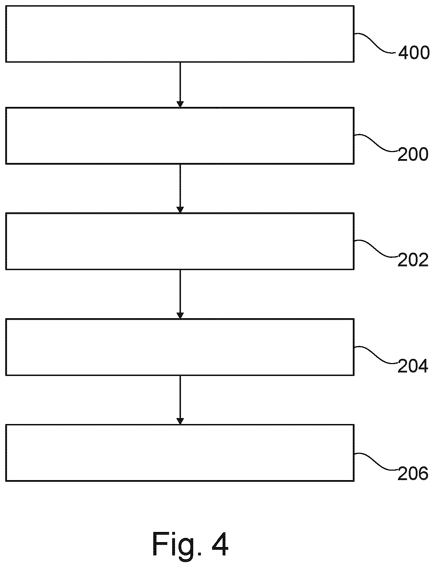

Fig. 4 shows a flowchart which illustrates a method of operating the medical system 300 of Fig. 3. In step 400, the X-ray system 302 is controlled to acquire the chest X-ray image 124. After step 400, steps 200, 202, 204, and 206 are performed as was illustrated in Fig. 2.

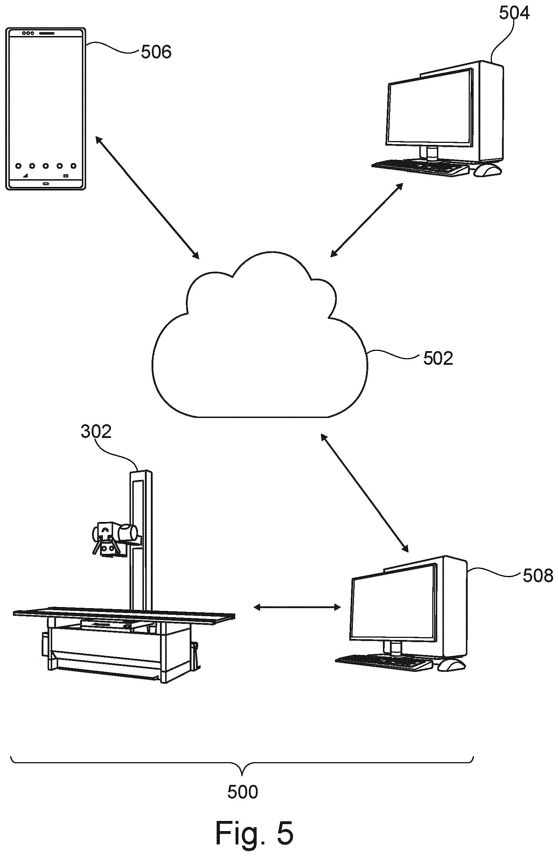

Fig. 5 illustrates a further example of the medical system 500. In this example the computational functionality of the computer 102, illustrated in Figs 1 and 3, are divided amongst a number of computational devices. There is a cloud computing system 502 that is connected to a workstation 504, a handheld computing device 506, and a local controller 508. The local controller 508 is used to control and operate the X-ray system or X-ray system 302. On this control, any of the steps illustrated in Figs. 2 and 4 may be distributed amongst the devices 502, 504, 506, and 508. In a typical usage the local controller 508 will control the X-ray system 302 to acquire the chest X-ray image 124 and then the cloud computing system 502 may produce or calculate the enhanced chest X-ray image 132. The enhanced chest X-ray image 132 may then be provided to the workstation 504 or the handheld computing device 506.

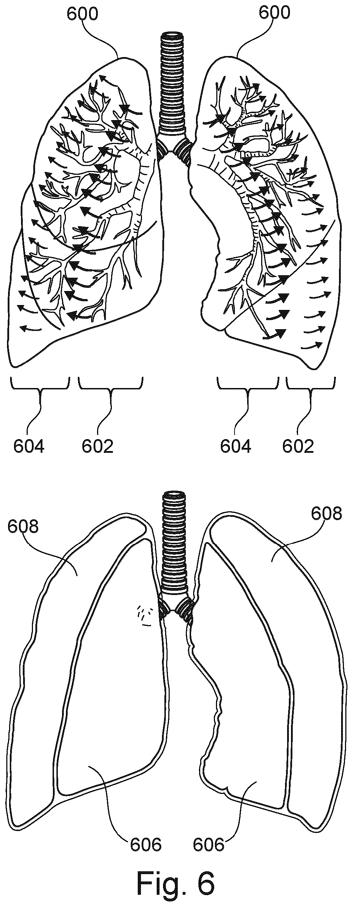

Fig. 6 shows an example of the lungs 600. One image shows a number of thicker arrows labeled 602, which indicate a higher blood density 602 and narrower arrows 604 which represent a lower blood density. As there are more branches in the interior section of the lung parenchyma than in the periphery, the blood density is more in the interior part of the lung than the periphery. The other image of the lungs 600 uses this and has divided the lungs into two regions, a higher X-ray attenuation region 606, which corresponds to the higher blood density 602 and a lower X-ray attenuation region 608, which corresponds to the lower blood density 604. The heart is located in a mid-region between the lobes of the lungs. The arteries and veins within the lungs are very large in the central region and then branch out into smaller veins and arteries.

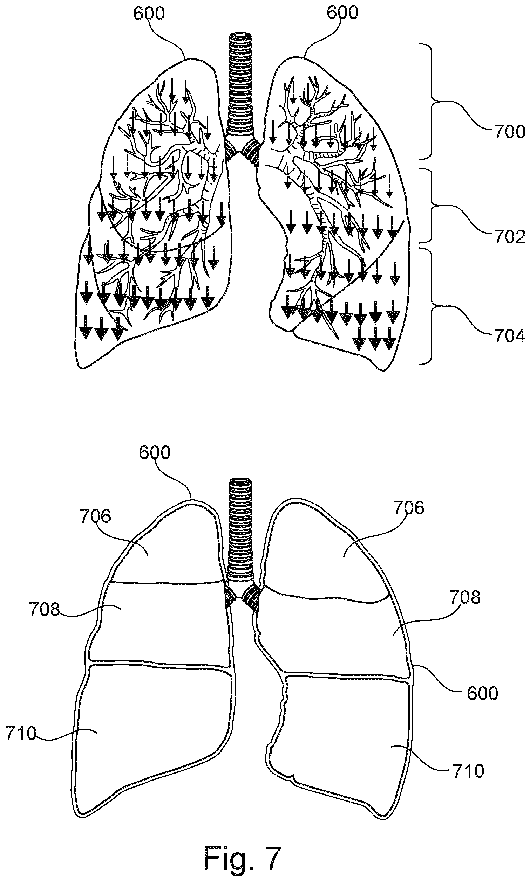

During chest radiograph procedure the patient stands in front of the detector. In this position, due to Earth's gravity more bloods get pulled towards the lower part of the lungs. Fig. 7 illustrates the effect of gravity on the blood density and the attenuation of X-rays in the lungs. The lungs are depicted and there are arrows which depict regions of a lower blood density region 700, a medium blood density region 702, and the higher blood density region 704. The higher blood density region 704 is at the lowest region because gravity pulls the blood to the base of the lungs. The low blood density region 700 results in a lower X-ray attenuation region 706. The medium blood density 702 results in a medium X-ray attenuation region 708. The higher blood density 704, caused by gravity results in a higher X-ray attenuation region 710. The effects illustrated in Figs. 6 and 7 combine to create the pulmonary blood density map 122.

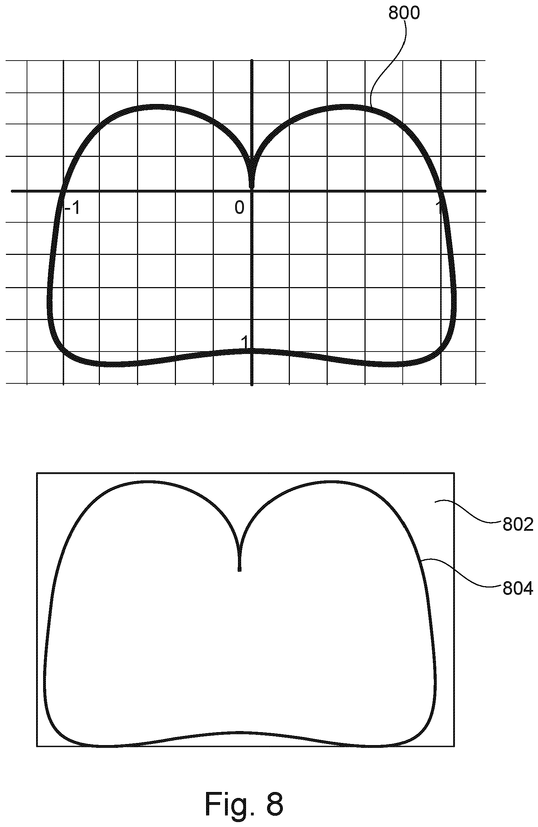

Fig. 8 shows the boundary of the pulmonary blood density map 122 as defined by a polynomial 800 in Eq. 1 below: ${y}^{8} + {x}^{2} {y}^{2} + {x}^{6} - {x}^{2} = - y \left({x}^{2}+{y}^{4}\right)$

This polynomial provides an effective approximation of the shape of lungs in a chest X-ray.

This same boundary is also shown as being rendered in an image 802. In this case, the boundary 804 has been rendered into the image 802. Fig. 8 illustrates that either an analytical boundary or one defined in an image may be used to construct the pulmonary blood density map 122.

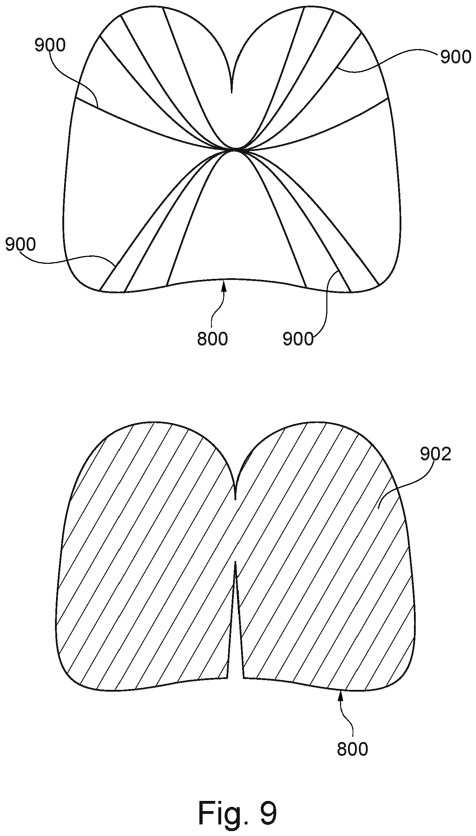

Fig. 9 shows two images. The first image shows the boundary 800 of the pulmonary blood density map 122 and a number of parabolas 900, which represent the arteries and veins and the branches inside of the lung. The second image shows the boundary 800 of the pulmonary blood density map 122 and the set of all possible parabolas 902.

This is used to model the branches (representation of the pulmonary arteries and veins) inside the pulmonary blood density map 122. To achieve that parabolas are drawn from each point of left half of the mask outline to the corresponding/mirror point of the right half. Each such parabola passes through the centroid of the mask, this point is the vertex for every parabola. These parabolas represent the branching of veins and arteries inside the lung.

A model to calculate and assign weights to the branches (of arteries and veins) based on the projective blood density is described:
A second mathematical model to calculate and assign weights to each point on each parabola inside the lung mask, based on these weights the low x-ray attenuation regions will be enhanced. The methodology is as follows:

First, an initial weight between 0 and 1 is assigned to each point on each parabola. The weight at the vertex of each parabola is assigned 0 and moving towards periphery (both the side of axis of symmetry) the points will be assigned with higher weights (within range 0 to 1). In this manner, the peripheral region of the mask will get respectively higher weights than the middle/inner region of the mask.

To improve the initial weight distribution, Eq 2 below is used to further magnify the weights in the peripheral regions of the mask: ${y}^{3} = a sin \left(0.000001+{πx}^{2}\right) ,$ where 0 ≤ x ≤ 1 and 1 ≤ a ≤ n and n is a positive number.

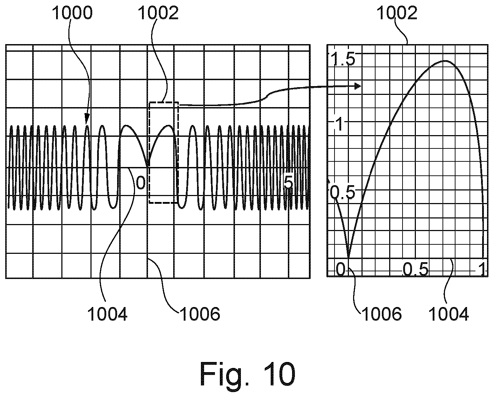

Fig. 10 shows a plot of equation 2 1000. The box 1002 shows the region of equation 2 used for the weight calculation of points on parabolas where 0 ≤ *x* ≤ 1 and 1 ≤ *a* ≤ n where n is a positive number... The X-axis is labeled 1004 and the Y-axis is labeled 1006.

In Equation 2, for a given value of *x* the value of *y* gets magnified, this magnification is high for a particular range of values of *x,* the constant '*a*' further magnify the values of *y.* So, one uses this equation to assign higher weights to the lung mask region corresponding to low attenuation areas in lungs (cf. Figs. 6 and 7). This will take care of the low attenuation of X-ray due to pulmonary blood circulation..

The constant '*a*' in the equation 2, which further increases the value of *y* for a given x. Constant '*a*' ranges from 1 to n. For higher values of '*a'* the magnification of *y*'s value is high, when '*a*' is 1 then the additional magnification is not there. When '*a*' has higher values, we have used the values of *y* as weights in parabolas from medium x-ray attenuation area towards low X-ray attenuation areas of lungs. As these two regions impacted by both pulmonary circulation (cf. Fig. 6) and factor of gravity (cf. Fig. 7). The weight distribution in the high X-ray attenuation area is based on *y*'s value when '*a*'=1, to take care of low attenuation in periphery due to only pulmonary blood circulation.

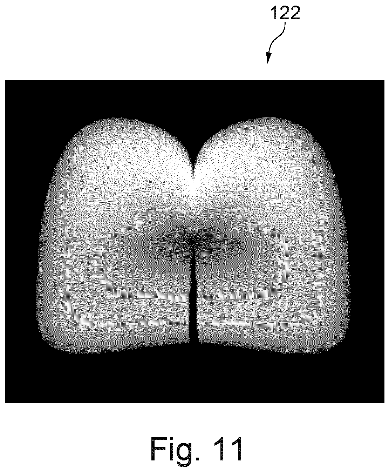

Fig. 11 shows a rendering of the pulmonary blood density map 122 rendered as a grayscale image.

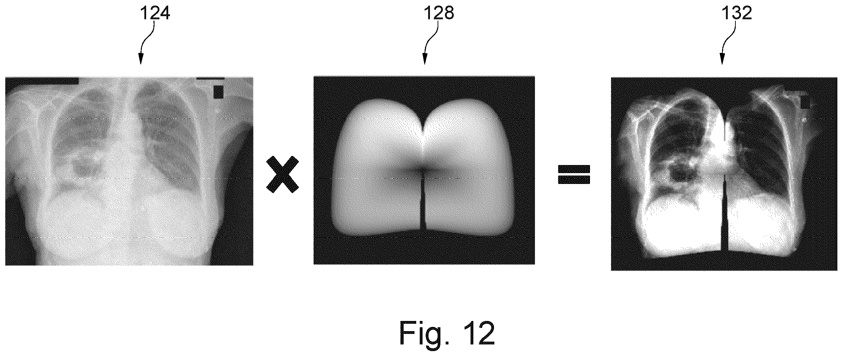

Fig. 12 illustrates the generation of an enhanced chest X-ray image 132. In this example the chest X-ray image 124 is multiplied voxel-wise with the registered pulmonary blood density map 128. This results in the enhanced chest X-ray image 132. In some cases the enhanced X-ray image 132 may be scaled so that it has the original bit range of the chest X-ray image 124.
Next, the mask is applied (refer Fig. 10, Fig. 11) to the chest x-ray image and the enhancement of the low-attenuation area due to difference in projective blood density in the original x-ray image is achieved.

The steps illustrated in Fig. 12 are as follows, from the chest X-ray 124, a bounding box containing the lung region is segmented, a CNN(Convolution Neural Network) based object detection model such as YOLO can be used for this method, alternatively lung edge detection based bounding box approach can also be performed to extract the lung region. Once the lung bounding box is extracted and resized according to the mask, the mask 128 is applied to the lung bounding box using a weighted multiplicative function and the result is rescaled to its original range of 8 bit(jpeg,png image) or 12 bit(medical Dicom image). The weight used during multiplication controls the amount of enhancement performed based on target lesion.

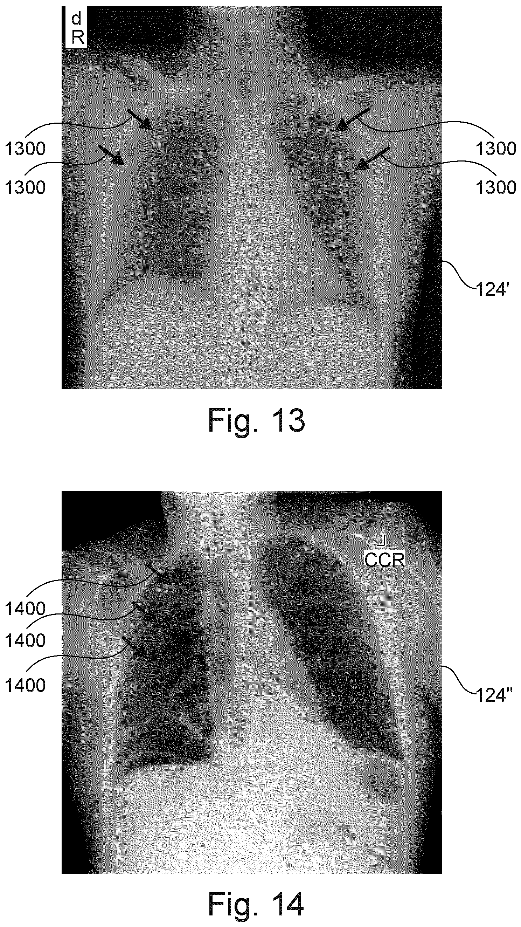

Fig. 13 shows a chest X-ray image 124' that shows ground glass opacity lesions 1300 which may be an indication that a subject has had COVID. In covid patients, GGO 1300 is observed as a gray opacification along the periphery of the lung parenchyma. As the blood density is less in the lung periphery region due to the structure of pulmonary artery and pulmonary veins and gravity effect, the x-ray attenuation is also lesser, which causes a poor visibility of lung lesion such a GGO in the peripheral region. Our proposed technique which considers and compensate low x-ray attenuation in lung periphery due to less blood, can be used to improve the appearance/visibility of GGO by considering. GGO is also indicative of other disease such as pneumonia.

Fig. 14 shows a further example of a chest X-ray image 124" that shows a pneumothorax image and may be used to indicate a collapsed lung. The arrows 1400 indicate the location of a visceral pleura which shows the lung in a state of partial collapse. Detecting mild to moderate Pneumothorax, where the lungs gets collapsed due to some injury etc. can be difficult. In chest x-ray image Pneumothorax appears as a linear shadow of visceral pleura with lack of lung markings peripheral to the shadow. Many times, such peripheral pneumothorax are hard to detect as the shadow of the visceral pleura is very thin. Examples may be able to enhance the lung periphery area and improve the visibility of the pneumothorax.

The enhancement of the pulmonary cavity region is discussed next. The inside section of a pulmonary cavity is darker than other lung region and more often there is a boundary (with dark to bright transition) surrounding the pulmonary cavity. Many times there are weak boundary/edges surrounding the pulmonary cavity. Based on this anatomy of the pulmonary cavity we identified the approximate pixel range that forms the ring surrounding the pulmonary cavity region. Next step is to further enhance this surrounding ring region so that during edge detection particularly the pulmonary cavity edges gets highlighted. Eq. 3 below is used to further enhance those pulmonary cavity edge having selected pixel range: ${y}^{3} = sin \left(0.000001+{aπx}^{2}\right) ,$ where 1 ≤ *a* ≤ n and 0 ≤ x ≤ 1, and n is a positive number.

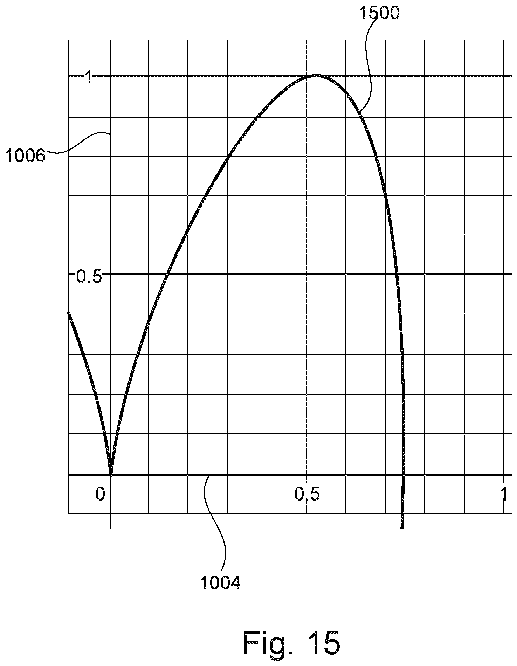

Fig. 15 shows a plot of equation 3 when a is between 1 and n and x is between 0 and 1. The X-axis is labeled 1004 and the Y-axis is labeled 1006.

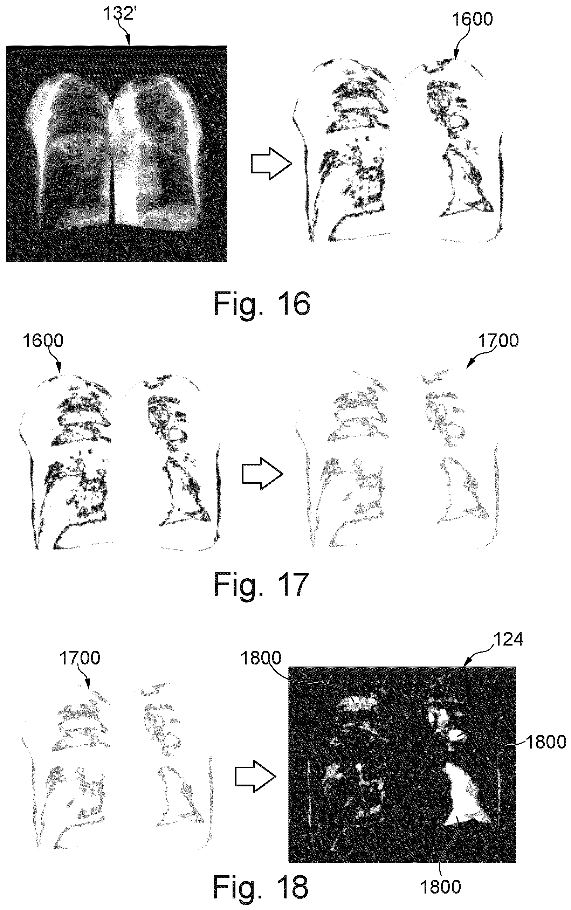

Figs. 16-23 illustrate the detection of lung cavities within a chest X-ray image 124. The pulmonary cavity lesions (also referred to as a "pulmonary cavity" herein) are an indication of tuberculosis. Fig. 16 illustrates pulmonary cavity edge enhancement. In this image an enhanced chest X-ray image 132' which shows pulmonary cavity lesions has an edge enhancement algorithm applied to it and results in an edge enhanced image 1600.

In Fig. 17 the edge enhanced image 1600 has an edge detection algorithm applied to it to produce an edge detection image 1700. Various enhancement and edge detection algorithms may be applied and the step shown in Fig. 16 may be optional, in some cases an edge detection algorithm may be applied directly to the enhanced chest X-ray image 132'.

In Fig. 18, the edge detection image 1700 is used with connected component analysis to identify the closed regions 1800 in either the chest X-ray image 124 or the enhanced chest X-ray image 132'.

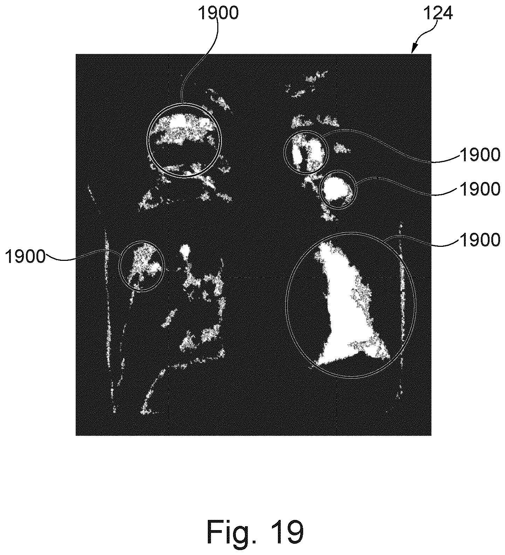

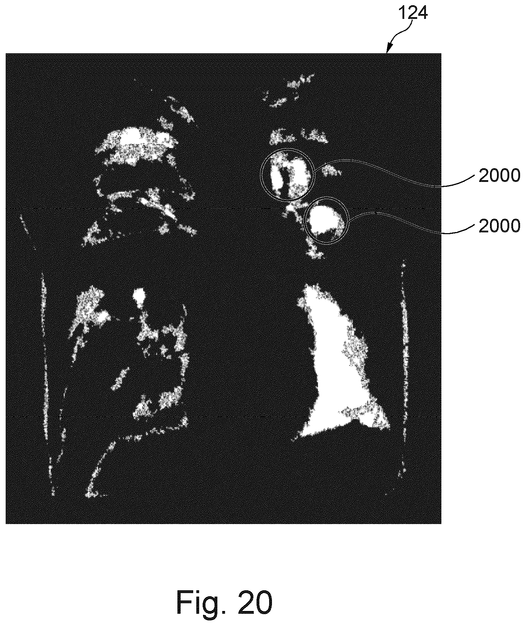

Fig. 19 illustrates the identification of connected components 1900 in the chest X-ray image 124 using the enclosed regions 1800 that were identified. In Fig. 20, the pulmonary cavity candidates 2000 are identified by using a pulmonary cavity detection algorithm applied to the connected components 1900.

In Fig. 20, two pulmonary cavity candidates 2000 are identified. The pulmonary cavity candidates 2000 are the connected components 1900 which have a more or less circular shape.

Once the pulmonary cavity candidates are identified from the x-ray image, the next task is to detect the actual cavities from those candidates. The basic shape of a pulmonary cavity is circular or near circular. We will use this feature to detect the actual cavities from the selected candidates. To achieve this, one may use a tomographic sinogram based shape analysis of each selected contour (pulmonary cavity candidate) and measure the circularity of that. The methodology is as follows:
- For each contoured polygon, tomography is performed at a regular angle Θ up to 360 degree.
- Next a sinogram is generated from this tomography
- Finally the contour is represented in the frequency domain.

After tomography, distance of the perimeter of object from annulus should be uniform for a circle. The standard deviation of the sinogram indicates that uniformity (thus circularity) of the tomographed object. Standard deviation value zero means the object is a perfect circle. Based on this, the contours whose sinogram has a standard deviation zero or near zero are identified as circular/near circular shaped cavities and those with much higher standard deviation are discarded. We can define a threshold-based selection to regulate the degree of circularity for a contour to be a pulmonary cavity. Some amount of false positive results can also be present.

Alternatively, the cavities can be detected and segmented by using segmentation neural network model such as U-Net, nnUNet, FCN. The chest x-ray images first pre-processed using our proposed enhancement technique, then annotation of pulmonary cavity regions can be performed. These enhanced images with annotations can be used as training dataset to train the segmentation model. At inference, a new image can be processed using the trained model and detect and segment the pulmonary cavity region. Similarly, bounding box based detection of pulmonary cavity can also be performed using YOLO algorithm, the YOLO model can be trained on annotated image data mentioned above and at inference the model can detect lung cavities in a unseen new image and provide a bounding box around it.

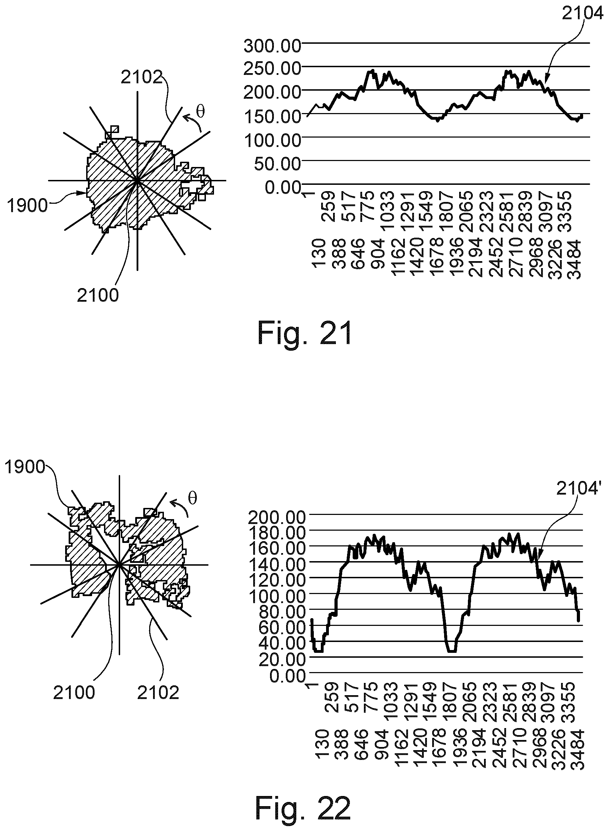

Fig. 21 illustrates the construction of a pulmonary cavity detection algorithm implemented using a tomographic sinogram algorithm. In this case there is a connected component 1900 and the central region 2100 has been identified using a centroid. Lines 2100 are drawn through the central region 2100 and used to calculate a tomographic or angular sinogram. This is then subjected to a spectral transform to calculate a frequency domain sinogram 2104. In Fig. 21 the shape is nearly circular and the standard deviation would be close to zero.

Fig. 22 shows a further connected component 1900 that is non-circular. The sinogram 2104' has a much larger standard deviation and indicates that the connected component 1900 is non-circular and is therefore not a pulmonary cavity candidate 2000.

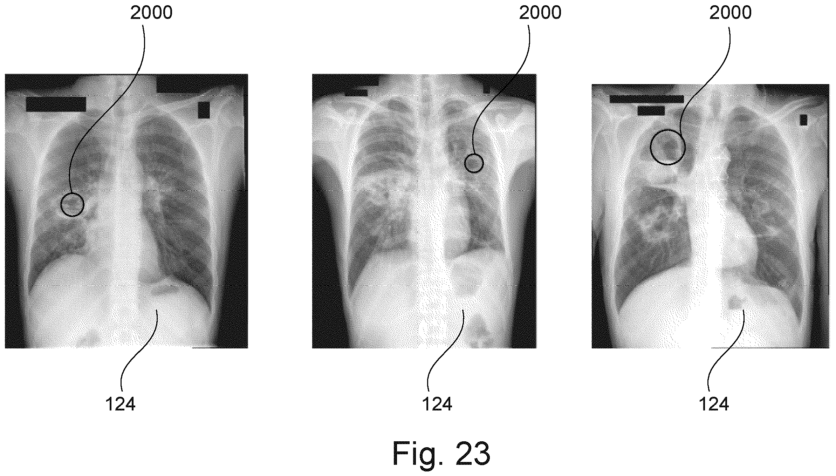

Fig. 23 shows examples of three different chest X-rays 124 that have had the method applied it and have each identified pulmonary cavity candidates 2000 within them.

It is understood that one or more of the aforementioned examples or embodiments of the invention may be combined as long as the combined embodiments are not mutually exclusive.

As will be appreciated by one skilled in the art, aspects of the present invention may be embodied as an apparatus, method or computer program product. Accordingly, aspects of the present invention may take the form of an entirely hardware embodiment, an entirely software embodiment (including firmware, resident software, micro-code, etc.) or an embodiment combining software and hardware aspects that may all generally be referred to herein as a "circuit", "module" or "system." Furthermore, aspects of the present invention may take the form of a computer program product embodied in one or more computer readable medium(s) having computer executable code embodied thereon.

Any combination of one or more computer readable medium(s) may be utilized. The computer readable medium may be a computer readable signal medium or a computer readable storage medium. A 'computer-readable storage medium' as used herein encompasses any tangible storage medium which may store instructions which are executable by a processor or computational system of a computing device. The computer-readable storage medium may be referred to as a computer-readable non-transitory storage medium. The computer-readable storage medium may also be referred to as a tangible computer readable medium. In some embodiments, a computer-readable storage medium may also be able to store data which is able to be accessed by the computational system of the computing device. Examples of computer-readable storage media include, but are not limited to: a floppy disk, a magnetic hard disk drive, a solid state hard disk, flash memory, a USB thumb drive, Random Access Memory (RAM), Read Only Memory (ROM), an optical disk, a magneto-optical disk, and the register file of the computational system. Examples of optical disks include Compact Disks (CD) and Digital Versatile Disks (DVD), for example CD-ROM, CD-RW, CD-R, DVD-ROM, DVD-RW, or DVD-R disks. The term computer readable-storage medium also refers to various types of recording media capable of being accessed by the computer device via a network or communication link. For example, data may be retrieved over a modem, over the internet, or over a local area network. Computer executable code embodied on a computer readable medium may be transmitted using any appropriate medium, including but not limited to wireless, wire line, optical fiber cable, RF, etc., or any suitable combination of the foregoing.

A computer readable signal medium may include a propagated data signal with computer executable code embodied therein, for example, in baseband or as part of a carrier wave. Such a propagated signal may take any of a variety of forms, including, but not limited to, electro-magnetic, optical, or any suitable combination thereof. A computer readable signal medium may be any computer readable medium that is not a computer readable storage medium and that can communicate, propagate, or transport a program for use by or in connection with an instruction execution system, apparatus, or device.

'Computer memory' or 'memory' is an example of a computer-readable storage medium. Computer memory is any memory which is directly accessible to a computational system. 'Computer storage' or 'storage' is a further example of a computer-readable storage medium. Computer storage is any non-volatile computer-readable storage medium. In some embodiments computer storage may also be computer memory or vice versa.

A 'computational system' as used herein encompasses an electronic component which is able to execute a program or machine executable instruction or computer executable code. References to the computational system comprising the example of "a computational system" should be interpreted as possibly containing more than one computational system or processing core. The computational system may for instance be a multi-core processor. A computational system may also refer to a collection of computational systems within a single computer system or distributed amongst multiple computer systems. The term computational system should also be interpreted to possibly refer to a collection or network of computing devices each comprising a processor or computational systems. The machine executable code or instructions may be executed by multiple computational systems or processors that may be within the same computing device or which may even be distributed across multiple computing devices.

Machine executable instructions or computer executable code may comprise instructions or a program which causes a processor or other computational systems to perform an aspect of the present invention. Computer executable code for carrying out operations for aspects of the present invention may be written in any combination of one or more programming languages, including an object-oriented programming language such as Java, Smalltalk, C++ or the like and conventional procedural programming languages, such as the C programming language or similar programming languages and compiled into machine executable instructions. In some instances, the computer executable code may be in the form of a high-level language or in a pre-compiled form and be used in conjunction with an interpreter which generates the machine executable instructions on the fly. In other instances, the machine executable instructions or computer executable code may be in the form of programming for programmable logic gate arrays.

The computer executable code may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a Local Area Network (LAN) or a Wide Area Network (WAN), or the connection may be made to an external computer (for example, through the Internet using an Internet Service Provider).

Aspects of the present invention are described with reference to flowchart illustrations and/or block diagrams of methods, apparatus (systems) and computer program products according to embodiments of the invention. It is understood that each block or a portion of the blocks of the flowchart, illustrations, and/or block diagrams, can be implemented by computer program instructions in form of computer executable code when applicable. It is further understood that, when not mutually exclusive, combinations of blocks in different flowcharts, illustrations, and/or block diagrams may be combined. These computer program instructions may be provided to a computational system of a general-purpose computer, special-purpose computer, or other programmable data processing apparatus to produce a machine, such that the instructions, which execute via the computational system of the computer or other programmable data processing apparatus, create means for implementing the functions/acts specified in the flowchart and/or block diagram block or blocks.

These machine executable instructions or computer program instructions may also be stored in a computer readable medium that can direct a computer, other programmable data processing apparatus, or other devices to function in a particular manner, such that the instructions stored in the computer readable medium produce an article of manufacture including instructions which implement the function/act specified in the flowchart and/or block diagram block or blocks.

The machine executable instructions or computer program instructions may also be loaded onto a computer, other programmable data processing apparatus, or other devices to cause a series of operational steps to be performed on the computer, other programmable apparatus or other devices to produce a computer implemented process such that the instructions which execute on the computer or other programmable apparatus provide processes for implementing the functions/acts specified in the flowchart and/or block diagram block or blocks.

A 'user interface' as used herein is an interface which allows a user or operator to interact with a computer or computer system. A 'user interface' may also be referred to as a 'human interface device.' A user interface may provide information or data to the operator and/or receive information or data from the operator. A user interface may enable input from an operator to be received by the computer and may provide output to the user from the computer. In other words, the user interface may allow an operator to control or manipulate a computer and the interface may allow the computer to indicate the effects of the operator's control or manipulation. The display of data or information on a display or a graphical user interface is an example of providing information to an operator. The receiving of data through a keyboard, mouse, trackball, touchpad, pointing stick, graphics tablet, joystick, gamepad, webcam, headset, pedals, wired glove, remote control, and accelerometer are all examples of user interface components which enable the receiving of information or data from an operator.

A 'hardware interface' as used herein encompasses an interface which enables the computational system of a computer system to interact with and/or control an external computing device and/or apparatus. A hardware interface may allow a computational system to send control signals or instructions to an external computing device and/or apparatus. A hardware interface may also enable a computational system to exchange data with an external computing device and/or apparatus. Examples of a hardware interface include but are not limited to: a universal serial bus, IEEE 1394 port, parallel port, IEEE 1284 port, serial port, RS-232 port, IEEE-488 port, Bluetooth connection, WLAN connection, TCP/IP connection, Ethernet connection, control voltage interface, MIDI interface, analog input interface, and digital input interface.

A 'display' or 'display device' as used herein encompasses an output device or a user interface adapted for displaying images or data. A display may output visual, audio, and or tactile data. Examples of a display include, but are not limited to: a computer monitor, television screen, touch screen, tactile electronic display, Braille screen, Cathode Ray Tube (CRT), storage tube, bi-stable display, electronic paper, vector display, flat panel display, Vacuum Fluorescent (VD) display, Light-Emitting Diode (LED) displays, Electro-Luminescent Display (ELD), Plasma Display Panels (PDP), Liquid Crystal Display (LCD), Organic Light-Emitting Diode (OLED) displays, a projector, and head-mounted display.

While the invention has been illustrated and described in detail in the drawings and foregoing description, such illustration and description are to be considered illustrative or exemplary and not restrictive; the invention is not limited to the disclosed embodiments.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measures cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

### REFERENCE SIGNS LIST

- 100: medical system
- 102: computer
- 104: computational system
- 106: optional hardware interface
- 108: optional user interface
- **110**: memory
- 120: machine executable instructions
- 122: pulmonary blood density map
- 124: chest X-ray image
- 124': enhanced chest X-ray image (ground glass opacity lesion)
- 124": enhanced chest X-ray image (pneumothorax image)
- 126: optional registration algorithm
- 128: registered pulmonary blood density map
- 130: optional product of chest X-ray image and registered pulmonary blood density map
- 132: enhanced chest X-ray image
- 132': enhanced chest X-ray image (pulmonary cavity)
- 200: receive a chest X-ray image
- 202: register a pulmonary blood density mask to the chest X-ray image
- 204: generate an enhanced chest X-ray image by compensating for a spatially dependent blood density in the chest X-ray image using the registered pulmonary blood density mask; and
- 206: provide the enhanced chest X-ray image
- 300: medical system
- 302: X-ray system
- 304: X-ray source
- 306: X-ray detector
- 308: subject
- 400: control an X-ray system to acquire the chest X-ray image
- 500: medical system
- 502: cloud computing system
- 504: work station
- 506: hand held computing device
- 508: local controller
- 600: lungs
- 602: higher blood density
- 604: lower blood density
- 606: higher X-ray attenuation region
- 608: lower X-ray attenuation region
- 700: lower blood density region
- 702: medium blood density region
- 704: higher blood density region
- 706: lower X-ray attenuation region
- 708: medium X-ray attenuation region
- 710: higher X-ray attenuation region
- 800: boundary of pulmonary blood density map defined by polynomial
- 802: image
- 804: boundary of pulmonary blood density map rendered as an image
- 900: parabola
- 902: all possible parabolas
- 1000: plot of equation 2
- 1002: region used for weight calculation
- 1004: X-axis
- 1006: Y-axis
- 1300: ground glass opacity legions
- 1400: visceral pleura
- 1500: plot of equation 3
- 1600: edge enhanced image
- 1700: edge detection image
- 1800: enclose region
- 1900: connected components
- 2000: pulmonary cavity candidates
- 2100: central region
- 2102: line
- 2104: frequency domain sinogram (circular object)
- 2104': frequency domain sinogram (non-circular object)

## Claims

1. A method of X-ray imaging comprising:
- receiving (200) a chest X-ray image (124, 124', 124");
- registering (202) a pulmonary blood density mask (122) to the chest X-ray image;
- generating (204) an enhanced chest X-ray image (132, 132') by compensating for a spatially dependent blood density (602, 604, 700, 702, 704) in the chest X-ray image using the registered pulmonary blood density mask (128); and
- providing (206) the enhanced chest X-ray image.

2. The method of claim 1, wherein generating the enhanced chest X-ray image by compensating for the spatially dependent blood density in the chest X-ray image using the registered pulmonary blood density mask comprises:
- performing pixel wise multiplication between the pulmonary blood density mask and the chest X-ray image; and
- preferably rescaling the enhanced chest X-ray image to an original bit range of the chest X-ray image.

3. The method of claim 1 or 2, wherein the pulmonary blood density mask is descriptive of a pulmonary blood density (602, 604) due to pulmonary blood circulation, and wherein the pulmonary blood density mask is preferably compensated for gravitational forces on the pulmonary blood circulation (700, 702, 704).

4. The method of claim 1, 2, or 3, wherein the pulmonary blood density map comprises an array of predetermined values.

5. The method of any one of the preceding claims, wherein the pulmonary blood density map has a boundary defined by a closed polynomial.

6. The method of any one of the preceding claims, wherein the pulmonary blood density map has weighting values defined by a mapping fit to parabolas passing through a central point of the pulmonary blood density map.

7. The method of claim 6, wherein the mapping is further adjusted using a gravitational factor.

8. The method of any one of the preceding claims, wherein the method further comprises identifying lung cavities in the enhanced chest X-ray image using a pattern recognition algorithm wherein the pattern recognition algorithm is configured to:
- generate an edge detection image (1700) by applying an edge detection algorithm to the enhanced X-ray image;
- detect enclosed regions (1800) in the edge detection image;
- identify connected components (1900) using the enclosed regions;
- identify pulmonary cavity candidates (2000) by applying a pulmonary cavity detection algorithm to the connected components; and
- provide the pulmonary cavity candidates.

9. The method of claim 8, wherein the pulmonary cavity detection algorithm, is any one of the following:
- a tomographic sinogram algorithm configured for identifying circular contours; and
- a trained pattern recognition neural network.

10. The method of claim 9, wherein the tomographic sinogram algorithm is configured to:
- receive a connected component selected (1900) from the connected components;
- identify a center location (2100) of the connected component;
- calculate an angular sinogram of the connected component which determines the density of the connected component along a line (2102) through the center location as a function of the angular rotation of the line;
- transform the angular sinogram to a frequency domain sinogram (2104, 2104") by spectrally transforming the angular sinogram;
- calculating a standard deviation of the frequency domain sinogram; and
- adding the connected component to the pulmonary cavity component if the standard deviation is below a predetermined threshold.

11. The method of any one of claims 1 through 7, wherein the enhanced X-ray image is any one of the following: a Pneumothrax detection image (124"), and a Ground Glass Opacity lesion detection image (124').

12. The method of any one of the preceding claims, wherein registering the pulmonary blood density mask to the chest X-ray image comprises any one of the following;
- fitting the pulmonary blood density mask to anatomical landmarks in the chest X-ray image; and
- fitting the pulmonary blood density mask to a bounding box receiving in response to inputting the chest X-ray image into a trained neural network.

13. The method of any one of the preceding claims, wherein the method further comprises controlling (400) an X-ray system (302) to acquire the chest X-ray image.

14. A computer program product comprising a computer readable storage medium (110) having machine executable instructions (120) embodied therewith, said machine executable instructions are configured to implement the method of any one of claims 1 through 13.

15. A medical system comprising:
- a memory (110) storing machine executable instructions (120) and a pulmonary blood density mask (122);
- a computational system (104), wherein execution of the machine executable instructions causes the computational system to:
- receive (200) a chest X-ray image (124, 124', 124");
- register (202) a pulmonary blood density mask (122) to the chest X-ray image;
- generate (204) an enhanced chest X-ray image (132, 132') by compensating for a spatially dependent blood density (602, 604, 700, 702, 704) in the chest X-ray image using the registered pulmonary blood density mask (128); and
- provide (206) the enhanced chest X-ray image.
